(19) Europäisches Patentamt
European Patent Office
Office européen des brevets

(11) Publication number: **0 086 363**
**B1**

(12) **EUROPEAN PATENT SPECIFICATION**

(45) Date of publication of patent specification: **24.04.85**

(21) Application number: **83100653.1**

(22) Date of filing: **25.01.83**

(51) Int. Cl.[4]: **C 07 C 83/02,** C 07 C 135/00, C 07 C 147/14, C 07 C 149/06, C 07 C 149/12

(54) **Process for the preparation of arylhydroxylamines.**

(30) Priority: **29.01.82 US 343994**

(43) Date of publication of application: **24.08.83 Bulletin 83/34**

(45) Publication of the grant of the patent: **24.04.85 Bulletin 85/17**

(84) Designated Contracting States: **AT BE CH DE FR GB IT LI NL SE**

(56) References cited:
**GB-A-1 320 270**
**GB-A-1 388 523**

**Chemical Abstracts vol. 91, no. 7, 13 August 1979, Columbus, Ohio, USA, T. TSURUTANI "Selective hydrogenation of aromatic nitro compounds", page 669, column 1, abstract 56604w**

(73) Proprietor: **MALLINCKRODT, INC.**
**675 McDonnell Boulevard P.O. Box 5840**
**St. Louis Missouri 63134 (US)**

(72) Inventor: **Caskey, Douglas C.**
**9 Belleau Lake Court**
**O'Fallon Missouri (US)**
Inventor: **Chapman, Douglas W.**
**48 Greendale Drive**
**St. Louis Missouri (US)**

(74) Representative: **Kraus, Walter, Dr. et al**
**Patentanwälte Kraus & Weisert Irmgardstrasse 15**
**D-8000 München 71 (DE)**

**EP 0 086 363 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

*Background of the Invention*

This invention relates to the field of catalytic hydrogenation of nitroaromatics and more particularly to an improved process for the selective preparation of arylhydroxylamine compounds by partial hydrogenation of the corresponding nitroaryl compounds.

Phenylhydroxylamine is produced by partial hydrogenation of nitrobenzene. Reaction of a mole of nitrobenzene with three moles of hydrogen in the presence of a platinum metal catalyst yields aniline:

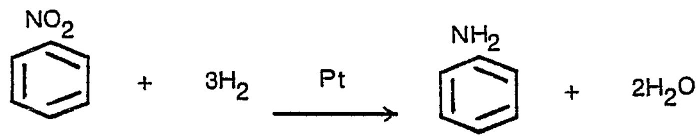


In an intermediate stage of the conversion of nitrobenzene to aniline, phenylhydroxylamine is produced upon reaction with two moles of hydrogen:

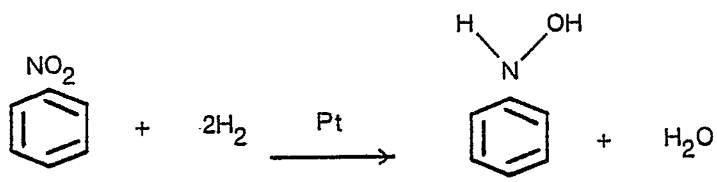


However, because the rate of conversion of phenylhydroxylamine to aniline is competitive with the rate of conversion of nitrobenzene to phenylhydroxylamine, the formation of aniline commences well before all of the nitrobenzene substrate is converted to the phenylhydroxylamine intermediate and, if hydrogen uptake is measured and reaction terminated after an uptake of two moles hydrogen per mole of nitrobenzene, approximately 20% of the product is typically aniline. If hydrogen introduction is terminated before the uptake reaches two moles per mole of nitrobenzene substrate, substantial proportions of unreacted nitrobenzene and other intermediates and byproducts are found in the reaction mixture, while partial conversion to aniline is not entirely eliminated.

Other arylhydroxylamines are produced by partial hydrogenation of corresponding nitroaryl compounds. Similar problems are encountered in significant conversion to the arylamine before all of the nitroaryl substrate can be converted to the arylhydroxylamine.

US—A—3,694,509 (Rylander et al.) describes a method for producing arylhydroxylamines while minimizing the fraction of arylamine produced prior to the uptake of two moles hydrogen per mole of substrate. Rylander et al. achieve this improvement by incorporating from 0.1 to 100 moles dimethyl sulfoxide per gram-atom of catalyst metal in the reactor charge mixture. However, while the Rylander et al. process affords a distinct improvement over previously known processes for the production of arylhydroxylamines, appreciable fractions of arylamine are still obtained and overall yields of arylhydroxylamine are still only modest. Moreover, in carrying out the Rylander et al. process, it is essential to terminate reaction when the hydrogen uptake reaches two mole per mole of nitroaryl substrate. If hydrogen introduction is not terminated at this point, reaction will continue and yields deteriorate. This circumstance detracts from the commercial value of the Rylander et al. process since accurate measurement of total hydrogen flow is difficult to achieve.

JP—A 54 24837—[1979] discloses a process for producing aryl hydroxylamines by catalytic hydrogenation of substituted or unsubstituted nitrobenzene in a solvent containing phosphoric acid, its alkali metal salts, an ester phosphite, an ester thiophosphite, an alkyl or aryl phosphine, an alkyl or aryl aminophosphine, an alkyl or aryl sulfide, a carboalkoxyalkyl sulfide, an alkyl or aryl mercaptan or thiophene.

However, a need has remained in the art for a further improved process of making phenylhydroxylamine from nitrobenzene and other arylamines from the corresponding nitroaryl compounds, both in high yield and with effective control of the reaction end point.

Because phenylhydroxylamine is an intermediate for other products such as N-nitrosophenylhydroxylamine, there has been a further need for a process by which phenylhydroxylamine or other arylhydroxylamines can be produced in high yield and thereafter converted *in situ* to the N-nitrosophenylhydroxylamine or other N-nitrosoarylhydroxylamines without recovery and purification of the intermediate. Conventionally, N-nitrosophenylhydroxylamine has been prepared using phenylhydroxylamine obtained by reduction of nitrobenzene with zinc dust.

*Summary of the Invention*

Among the several objects of the present invention, therefore, may be noted the provision of an improved process for the manufacture of phenylhydroxylamine and other arylhydroxylamines; the provision of such a process which produces phenylhydroxylamine in high and improved yields; the provision of such a process which minimizes conversion to the arylamine; the provision of such a process whose end point can be readily controlled and identified to prevent introduction of excessive amounts of hydrogen; the provision of such a process which enhances the selectivity of the catalyst in maximizing yields of any hydroxylamines; the provision of such a process which produces the arylhydroxylamine in a reaction mixture to which other reagents can be added and the arylhydroxylamine reacted therewith *in situ* without prior recovery or purification of the hydroxylamine intermediate; and in particular, the provision of such a process by which an N-nitrosoarylhydroxylamine can be produced.

Briefly, therefore, the present invention is directed to a novel process for producing an arylhydroxylamine having the formula

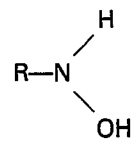

where R is aryl or substituted aryl, in high yield and with minimal formation of by-product arylamine. In the process, a charge mixture is prepared comprising a nitroaryl having the formula:

$$R{-}NO_2$$

where R is defined above, a catalyst containing platinum, a base, a sulfur compound, and solvent for the nitroaryl compound. The sulfur compound is either a divalent sulfur compound in which sulfur is bonded to two other moieties or a compound reducible to such divalent sulfur compound under catalytic hydrogenation conditions. The base is ammonia, an aliphatic amine, phosphine, and aliphatic or aryl phosphine, or a phosphite ester. Alcohols, ethers, and esters may comprise the solvent. Hydrogen is introduced into the charge mixture while the mixture is agitated at a temperature in the range of between about 0 and about 40°C, thereby converting the nitroaryl compound to the arylhydroxylamine.

Other objects and features will be in part apparent and in part pointed out hereinafter.

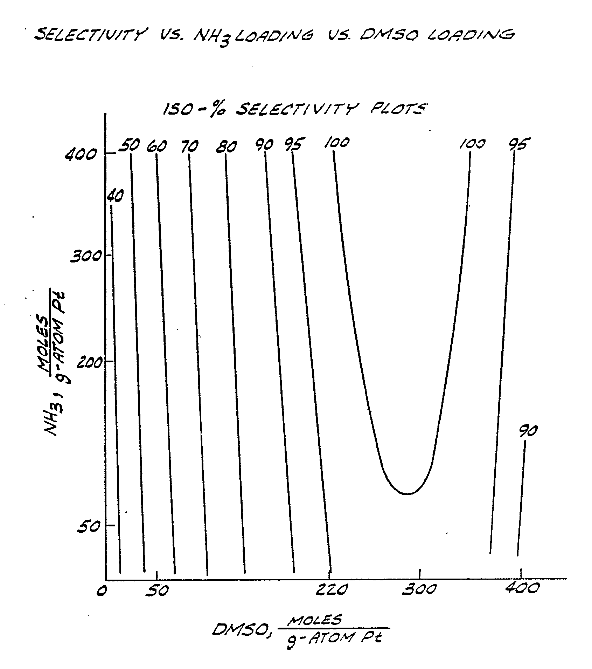

*Brief Description of the Drawing*

The single view of the drawing is an exemplary plot of catalyst selectivity contours for different combinations of dimethyl sulfoxide and ammonia in the solvent system hydrogenation of nitrobenzene to phenylhydroxylamine.

*Description of the Preferred Embodiments*

In accordance with the present invention, it has been found that the presence of certain bases increases the selectivity of the catalyst used in the catalytic hyodrogenation of nitroaryl compounds and retards the activity of the catalyst for conversion to the arylamine of the arylhydroxylamine produced in the reaction. It has further been discovered that the combination of a sulfur compound, such as dimethylsulfoxide, and base affords further improvement in the selectivity of the catalyst and essentially eliminates all conversion of the hydroxylamine to the amine. Because conversion to the arylamine is essentially eliminated, hydrogen uptake is spontaneously and completely terminated after the addition of two moles of hydrogen, thereby providing for both control and identification of the reaction end point, and greatly facilitating the practical implementation of the process in plant operations.

Generally, therefore, the method of the invention is effective for obtaining high yields of arylhydroxylamines having the formula

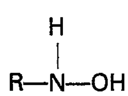

where R is aryl or substituted aryl, by partial catalytic hydrogenation of nitroaryl compounds having the formula

$$R{-}NO_2$$

where R is as defined above. Typical nitroaryl substrates which may be used in the process of the invention include nitrobenzene, o-nitrotoluene, m-nitrotoluene, p-nitrotoluene, p-nitrophenol 2,6-dimethylnitrobenzene and alpha-nitronaphthalene. A wide variety of other nitroaryls may also be used.

In accordance with the invention, it has further been determined that sulfur compounds other than the dimethyl sulfoxide are useful agents for enhancing catalyst selectivity and retarding conversion of the

hydroxylamine to the amine. Generally, it has been discovered that the most effective sulfur compounds are divalent sulfur compounds in which sulfur is bonded to two other moieties, particularly dialkyl sulfides such as dimethyl sulfide, ethyl propyl sulfide, bis(dodecyl)sulfide and methyl octyl sulfide. While we do not wish to be bound to a particular theory, it further appears that sulfoxides such as dimethylsulfoxide may not be especially effective per se, but become efficient selectivity enhancing agents when converted to the divalent sulfur compound (i.e. the sulfide) under catalytic hydrogenation conditions. Generally, therefore, the sulfur compound may be either the aforesaid divalent compound in which sulfur is bonded to two other moieties or a compound, such as a sulfoxide, which is reducible to a divalent sulfur compound under the aforesaid conditions. Although dialkyl sulfides are most preferred, other compounds such as diaryl sulfides, aryl alkyl sulfides, disulfides, thiols, thiophene, thiophane and hydrogen sulfide may be successfully used.

Platinum is used as the catalyst for the partial hydrogenation reaction of this invention. Preferably, the catalyst is provided in metallic form on a carbon support at a level of approximately 0.5 to about 5% by weight platinum. Alternatively, but less preferably, the platinum can be supplied on another carrier such as alumina or kieselguhr. Platinum oxide catalysts can also be used, but platinum metal is preferred. Catalyst loading is also a parameter of some importance in the process of the invention. For the reduction of nitrobenzene to phenylhydroxylamine, catalyst loading is preferably in the range of between $3.0 \times 10^{-5}$ to $5.0 \times 10^{-4}$ gram-atom platinum per mole of nitrobenzene substrate. For other nitroaryl reductions, the loading is approximately the same as for nitrobenzene, but the optimum may vary slightly and can be readily determined by routine experimentation.

Where a dialkyl sulfoxide such as dimethyl sulfoxide is used as the sulfur compound in the charge mixture, it has been found that best results are achieved at a concentration greater than 100 moles per gram-atom platinum metal. Generally, the sulfoxide compound concentration should fall in the range of between 150 and 400 moles per gram atom catalyst, with approximately 220—260 moles per gram-atom being most preferred.

Where a dialkyl sulfide, aryl alkyl sulfide, thiol, or other divalent sulfur compound is used for catalyst modifications, it has been found that the required dosages are much lower than in the case of a sulfoxide. It is this finding which suggests that the sulfide is the operative agent for control of catalyst activity and selectivity and that the sulfoxides are effective to the extent that they are converted to the sulfide under catalytic hydrogenation conditions. Thus, where a sulfide is initially supplied to the charge mixture, its range of concentrations and optimum concentration are less than 5% of those specified above for the sulfoxides, i.e. between 0.1 and 10 moles per gram-atom of platinum.

As noted above, it has further been discovered that significant improvement in catalyst selectivity and end point control are provided by the use of a basic medium rather than the neutral medium taught by Rylander, et al. However, not all bases are of equal value in creating favorable conditions for the partial hydrogenation reaction. Thus, ammonia and aliphatic amines are strongly preferred. Primary, secondary, and tertiary amines can all be used. Phosphite esters such as triethyl and triphenyl phosphite are suitable but inorganic bases generally are not. Phosphine and both aliphatic and arylphosphines can also be used but are preferably avoided because of their toxicity. Arsines are also believed to be effective but are extremely toxic and would normally not be used.

Because of the cooperative effect of the sulfur compound and the Lewis base, the optimum proportion of base varies with the proportion of sulfur compound in the charge mixture. Generally it is preferred that the mixture contain between 100 and 300 moles of base per gram atom of platinum in the mixture. At most sulfur compound concentrations, further increase in base concentration have a positive but relatively slight effect on selectivity. However, there is typically an optimum proportion of sulfur compound at which essentially 100% selectivity can be achieved at only a very modest base concentration, for example, 50—100 moles per gram-atom of platinum (see the drawing). Because selectivity is adversely affected by sulfur compound concentrations in excess of the optimum, the relationship between sulfur compound and base makes it possible and desirable to adopt a process control strategy in which sulfur compound is charged to a level short of the optimum and base additive is used as a vernier to adjust to a combination that provides optimum performance. Some excess of base has no adverse effect on selectivity. Moreover, in the case of ammonia, any significant excess can be removed by degassing the batch through inert gas purging or application of vacuum.

As the solvent for the charge mixture, methanol is preferred but other lower alcohols are satisfactory. Ethers, including cyclic ethers such as tetrahydrofuran are generally useful, as are carboxylic acid esters. While the use of a solvent is essential to prevent precipitation of the arylhydroxylamine from the reaction mixture, the process is preferably operated with a charge mixture that produces a reaction mixture close to the point of saturation, so as to maximize reactor payload and reduce both solvent losses and the energy and other operational costs of recovery.

In carrying out the method of the invention, a hydrogenator is charged with catalyst, nitroaryl compound, solvent, sulfur compound, and base. The reactor is thereafter purged with nitrogen or other inert gas to displace oxygen from the headspace, and then purged with hydrogen to eliminate the inert gas. After purging is complete, hydrogen pressure is increased, typically to 4.4—6.2 bar, and agitation commenced to initiate the reaction.

Reaction may be carried out at a temperature in the range of 0 to 40°C, but it is strongly preferred that

the reaction temperature be maintained below 20°C in order to maximize yield. It is also preferred that the reaction temperature not be lower than 15°C because at lower temperatures the reaction rate is quite slow, with an attendant penalty not only in productivity but also to some extent in yield, due to the longer exposure of reaction products to conditions under which they may be degraded or converted to various by-products. Depending on the concentration of nitroaryl substrate in the charge mixture, there is also some risk of precipitation of arylhydroxylamine product on the catalyst at reaction temperatures below 15°C.

Hydrogen pressure is not a critical variable but high pressures do accelerate the reaction rate, reduce the cycle time and concomitantly reduce the time during which reaction products are exposed to conditions under which they may be converted to by-products or degrade. No adverse effects, such as ring saturation reactions, have been observed up to pressures of 12.0 bar. Generally, a hydrogen pressure in the range of 2.7—14.8 bar is considered suitable. In order to maximize the reaction rate at a given available hydrogen pressure, it is desirable to provide for both maximum feasible cooling capacity and vigorous agitation since hydrogen introduction is normally limited by heat removal capacity during the early portion of the cycle, and the reaction rate is limited by mass transfer rates during the later portion.

In contrast to prior commercial methods for producing arylhydroxylamines by partial hydrogenation of nitroaryl compounds, the process of the invention does not require close measurement of total hydrogen flow in order to determine the end point of the reaction. By use of the combination of a base and a sulfur compound of the above noted character, selectivity is improved to the extent that conversion of the hydroxylamine to the arylamine is essentially precluded. Thus, not only is the yield of hydroxylamine high, but the reaction stops spontaneously and the consequent termination in hydrogen flow marks the end point of the reaction. Provision of a hydrogen flow indicator is desirable to both monitor reaction progress and detect the cessation of hydrogen flow.

After the reaction is complete, the reaction mixture is filtered to remove the catalyst and, if desired, the arylhydroxylamine can be recovered by conventional techniques. Thus, for example, a portion of the solvent may be driven off by evaporation at an elevated temperature after which the solution may be cooled to separate the arylhydroxylamine by crystallization.

As noted, arylhydroxylamines are useful intermediates in making other products such as for example N-nitrosoarylhydroxylamines. A distinct advantage of the process of the invention is the feasibility of converting the hydroxylamine to such further products without prior recovery of the hydroxylamine from the reaction mixture. In the case of the preparation of the N-nitroso compound, anhydrous ammonia is introduced into the filtered arylhydroxylamine liquor to provide and maintain an excess of ammonia, typically at a concentration of at least 0.0059868 kg/l (0.06 lbs/gal). Temperature of the arylhydroxylamine liquor should be maintained in the range of between 15 and 18°C during the addition of ammonia.

After the desired level of ammonia has been reached, as determined by laboratory analysis of a sample of the ammoniated liquor, nitrosation is commenced by the addition of an alkyl nitrite such as isopropyl nitrite to the liquor. Temperature during nitrite addition is maintained at 25°C or lower and sparging of ammonia is continued during the addition of the nitrite. Reaction between the nitrite and the phenylhydroxylamine produces the ammonium salt of the N-nitroso compound:

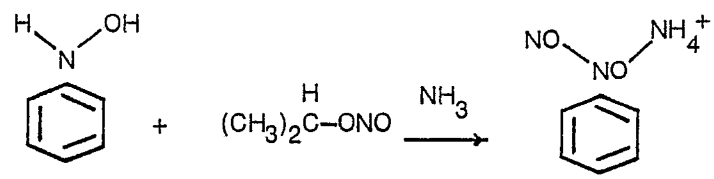

After introduction of an amount of alkyl nitrite equivalent to the arylhydroxylamine content of the liquor, addition of the nitrite compound and sparging of the ammonia are terminated and the nitrosation reaction mixture cooled to a temperature in a range of 0—10°C. To complete the reaction, the mixture is stirred for approximately an hour after the completion of nitrite addition, and then centrifuged for recovery of the N-nitroso compound. The centrifuged cake is washed with alcohol and the washed cake product is dried under a vacuum. In order to maintain the stability of the product, drying is carried out in the presence of ammonium carbonate.

Solvent may be recovered by distillation of the centrifuge mother liquor filtrate. Before distillation, the filtrate is treated with an aqueous caustic solution.

The following examples illustrate the invention.

## Example 1

Phenylhydroxylamine was prepared by catalytic hydrogenation of nitrobenzene in a stirred 1 liter steel pressure reactor having an automatic temperature controller. Temperature of the reaction in the pressure reactor was monitored by a thermocouple and cooling effected by cooling water passed through internal coils. A heating mantle was provided for heating of the contents of the reactor when necessary.

A reaction charge mixture was prepared by adding to the reactor nitrobenzene (150 ml), 3% platinum

on carbon catalyst (5.55 grams; 70% wet) methanol solvent (250 ml), dimethyl sulfoxide (4.00 grams) and 30% aqueous ammonia (1.35 grams).

After charging was completed the reactor was purged with nitrogen, then with hydrogen and pressurized with hydrogen to 10.6 bar. Reaction was commenced by starting of the agitator and temperature was controlled during the reaction in a range of 18 to 20°C. Hydrogen uptake was monitored with a rotameter and hydrogen absorption terminated after slightly more than 2.00 moles hydrogen per mole nitrobenzene was consumed. At this point the hydrogen uptake stopped spontaneously and abruptly, signalling the end of the reduction reaction.

After the reaction was terminated, the reactor was purged with nitrogen, the reaction mixture was removed from the reactor and the catalyst was separated from the reaction mixture by filtration.

Example 2—16

Using the apparatus and method generally described in Example 1, a series of runs was made in which phenylhydroxylamine was produced by catalytic hydrogenation of nitrobenzene in the presence of a sulfur compound and a base. For each run, the time of the hydrogenation reaction was recorded and the selectivity was calculated. Selectivity is defined as the moles of phenylhydroxylamine produced divided by the moles of nitrobenzene reacted times 100. Results of the runs of these Examples are set forth in Table 1.

TABLE 1

| Example # | moles Modifier g-atom Pt | Catalyst wgt., g. | Pressure, $H_2$ bar | Time, min. | Selectivity |
|---|---|---|---|---|---|
| 2 | 68 DMSO, 94 $NH_3$ | 1.39 g | 7.9 | 205 | 81.4 |
| 3 | 68 DMSO, 94 $NH_3$ | 2.78 | 7.9 | 115 | 87.5 |
| 4 | 136 DMSO, 185 $NH_3$ | 2.78 | 7.9 | 128 | 88.4 |
| 5 | 200 DMSO, 185 $NH_3$ | 2.78 | 7.9 | 140 | 90.7 |
| 6 | 400 DMSO, 185 $NH_3$ | 2.78 | 10.6 | 120 | 92.4 |
| 7 | 400 DMSO | 2.78 | 7.9 | 160 | 89.3 |
| 8 | 136 DMSO, 186 $NH(CH_3)_2$ | 2.78 | 7.9 | 127 | 90.9 |
| 9 | 136 DMSO | 2.78 | 7.9 | 116 | 80.5 |
| 10 | 10 $S(CH_3)_2$, 185 $NH_3$ | 2.78 | 7.9 | 128 | 92.4 |
| 11 | 158 $S(CH_3)_2$, 185 $NH_3$ | 2.78 | 7.9 | 183 | 91.7 |
| 12 | 1.4 $S(CH_3)_2$, 185 $NH_3$ | 2.78 | 7.9 | 85 | 84.2 |
| 13 | 1.0 $S(C_2H_5)_2$, 185 $NH_3$ | 2.78 | 7.9 | 91 | 84.4 |
| 14 | 0.2 $CH_3CH_2CH_2CH_2SH$ 185 $NH_3$ | 2.78 | 7.9 | 165 | 77.6 |
| 15 | .09 $(CH_3)CHSSCH(CH_3)_2$ 185 $NH_3$ | 2.78 | 7.9 | 187 | 86.4 |
| 16 | 136 DMSO, 186 $N(C_2H_5)_3$ | 2.78 | 7.9 | 127 | 90.9 |

Example 17

Using the apparatus and method described in Example 1, a series of runs were made in which phenylhydroxylamine was produced by catalytic hydrogenation of nitrobenzene in the presence of dimethyl sulfoxide (DMSO) and ammonia. Proportions of DMSO and ammonia were varied to establish a statistical basis for determining catalyst selectivity as a function of the two catalyst modifying components. Data obtained from this series of runs was subjected to mathematical analysis which yielded a series of catalyst selectivity contours on an ammonia versus DMSO grid. These contours are plotted in the drawing.

It may be seen that plots such as the drawing provide a basis for process control through a strategy of

establishing a base level of DMSO and adjusting ammonia level. Catalyst selectivity is maximized at an optimum locus of combinations of DMSO and base represented by the contour labelled 100% in the drawing. Typically, DMSO is incorporated in the charge mixture at a level lower than what the optimum sulfur compound content would be at zero ammonia content, and the ammonia content adjusted to provide a combination on or near the optimum locus. In the instance of the drawing, approximately 200—260 moles DMSO per gram atom of platinum may be advantageously incorporated in the charge mixture and the ammonia level adjusted to between about 50 and about 300 moles per gram atom to achieve a combination on or near the aforesaid locus.

Example 18

The phenylhydroxylamine reaction mixture obtained in Example 1 was placed in a flask fitted with a stirrer and thermometer and surrounded by an ice water bath. Anhydrous ammonia gas was sparged into the mixture so that the mixture was maintained strongly basic. Ammonia concentration was maintained at 0.007 grams per ml or higher. Isopropyl nitrite (143 grams; 10% molar excess) was added slowly to the mixture in the flask at such rate as to maintain the temperature in the range of 10 to 20°C. Reaction between the phenylhydroxylamine and the isopropyl nitrite in the basic medium produced the ammonium salt of N-nitrosophenylhydroxylamine. After the nitrosation reaction was complete, the N-nitrosophenylhydroxylamine salt was recovered by filtration, dried and weighed. Based on the weight of the isolated product and considering solubility losses a selectivity of 95% was obtained.

Example 19

To a stainless steel one liter pressure reactor were charged:

o-nitrotoluene (116 g; 0.85 gram-moles)
methanol (300 ml)
dimethyl sulfoxide (1.34 g; 0.0172 moles)
concentrated aqueous ammonia (1.35 g; 0.0238 moles)
3% platinum on carbon catalyst, 70% wet (2.78 g)

Temperature of the charge mixture was adjusted to 15—20°C after which the reactor was sealed, purged with nitrogen and then purged with hydrogen. After purgation, the reactor was pressurized with hydrogen to 7.9 bar and agitation commenced at a rapid rate to start the reaction. During the reaction, the temperature of the reacting mixture was maintained in the range of 15—20°C by passage of cooling water through internal coils in the reactor. After slightly more than 2.00 moles hydrogen per mole of o-nitrotoluene had been absorbed, the hydrogen flow rate dropped essentially to zero. At this point, the reactor was purged with nitrogen and disassembled. The reaction mixture was then filtered to recover the catalyst and the o-methylphenylhydroxylamine solution filtrate was treated in the same manner as described in Example 18 to convert the o-methylphenylhydroxylamine to N-nitroso-o-methylphenylhydroxylamine. A selectivity of about 85% of theory was realized.

In view of the above, it will be seen that the several objects of the invention are achieved and other advantageous results attained.

As various changes could be made in the above methods without departing from the scope of the invention, it is intended that all matter contained in the above description or shown in the accompanying drawings shall be interpreted as illustrative and not in a limiting sense.

**Claims**

1. A process for producing an arylhydroxylamine having the formula

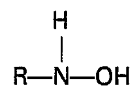

where R is aryl or substituted aryl, in high yield and with minimal formation of by-product arylamine compounds, the process comprising the steps of:
preparing a charge mixture comprising a nitroaryl compound having the formula

$R—NO_2$

where R is as defined above, a catalyst containing platinum, a base selected from the group consisting of ammonia, aliphatic amines, phosphine, aliphatic phosphines, aryl phosphines and phosphite esters, a sulfur compound selected from the group consisting of divalent sulfur compounds in which sulfur is bonded to two other moieties and compounds reducible to such divalent sulfur compounds under catalytic hydrogenation conditions, and a solvent for the nitroaryl compound, said solvent being selected from the group consisting of alcohols, ethers and esters; and

introducing hydrogen into said mixture while agitating the mixture at a temperature in the range of between 0 and 40°C, thereby reducing the nitro group of the nitroaryl compound to the hydroxylamine group.

2. A process as set forth in claim 1 wherein said sulfur compound is selected from the group consisting of sulfides and sulfoxides.

3. A process as set forth in claim 2 wherein said sulfur compound is selected from the group consisting of dialkyl sulfoxides and aryl alkyl sulfoxides.

4. A process as set forth in claim 3 wherein said sulfur compound comprises a dialkyl sulfoxide and said mixture contains between 150 and 400 moles of said dialkyl sulfoxide per gram atom of platinum.

5. A process as set forth in claim 2 wherein said sulfur compound is selected from the group consisting of dialkyl sulfides, dialkyl disulfides, aryl alkyl sulfides, hydrogen sulfide, thiols, thiophene, and thiophane.

6. A process as set forth in claim 1 wherein said sulfur compound comprises a dialkyl sulfide and said mixture contains between 0.1 and 10 moles of said dialkyl sulfide per gram atom of platinum.

7. A process as set forth in claim 1 wherein said base is selected from the group consisting of ammonia and aliphatic amines.

8. A process as set forth in claim 1 wherein said mixture contains between $3.0 \times 10^{-5}$ and $5.0 \times 10^{-4}$ gram-atoms platinum per mole of said nitroaryl compound.

9. A process as set forth in claim 4 or 8 wherein said base is selected from the group consisting of ammonia and aliphatic amines and said mixture contains between 100 and 300 moles of base per gram atom of platinum.

10. A process as set forth in claim 3 wherein there is a maximum catalyst selectivity at an optimum locus of combinations of sulfur compound content and base content, and the process is controlled by incorporating sulfur compound in the charge mixture at a level lower than the optimum sulfur compound content at zero base content, and adjusting the base content to provide a combination on or near said locus.

11. A process as set forth in claim 10 wherein said sulfur compound is dimethyl sulfoxide and said base is ammonia, the dimethyl sulfoxide is incorporated in the charge mixture at between 200 and 260 moles per gram atom of platinum, and the ammonia level is adjusted to between 50 and 300 moles per gram atom of platinum.

12. A process as set forth in claim 1 wherein said nitroaryl compound is selected from the group consisting of nitrobenzene, o-nitrotoluene, m-nitrotoluene, p-nitrotoluene, p-nitrophenol, 2,6-dimethylnitrobenzene and alpha-nitronaphthalene.

13. A process as set forth in claim 12 wherein said nitroaryl compound comprises nitrobenzene.

14. A process as set forth in claim 1 wherein said solvent comprises a primary alcohol.

15. A process as set forth in claim 1 wherein hydrogen is introduced into said mixture while the mixture is agitated at a temperature in the range of between 15 and 20°C.

16. A process as set forth in claim 1 wherein said arylhydroxylamine is converted to the ammonium salt of an N-nitrosoarylhydroxylamine by the steps of:

separating said catalyst from the reaction mixture obtained by hydrogenation of said charge mixture;

adding ammonia to said reaction mixture; and

adding an alkyl nitrite to said reaction mixture in the presence of ammonia, thereby converting the arylhydroxylamine to the ammonium salt of N-nitrosarylhydroxylamine.

17. A process as set forth in claim 1 wherein said sulfur compound comprises dimethyl sulfoxide.

18. A process as set forth in claim 5 wherein said sulfur compound is selected from the group consisting of dimethyl sulfide, ethyl propyl sulfide, bis(dodecyl) sulfide and methyl octyl sulfide.

19. A process for producing an arylhydroxylamine having the formula

$$\begin{array}{c} H \\ | \\ R—N—OH \end{array}$$

where r is aryl or substituted aryl, in high yield and with minimal formation of by-product arylamine compounds, the process comprising the steps of:

preparing a charge mixture comprising a nitroaryl compound having the formula

$$R—NO_2$$

where R is as defined above, a catalyst containing platinum, a base selected from the group consisting of ammonia, aliphatic amines, phosphine, aliphatic phosphines, aryl phosphines and phosphite esters, a divalent sulfur compound in which sulfur is bonded to two other moieties, and a solvent for the nitroaryl compound, said solvent being selected from the group consisting of alcohols, ethers and esters; and

introducing hydrogen into said mixture while agitating the mixture at a temperature in the range of between 0 and 40°C, thereby reducing the nitro group of the nitroaryl compound to the hydroxylamine group.

20. A process as set forth in claim 19 wherein there is a maximum catalyst selectivity at an optimum

locus of combinations of sulfur compound content and base content, and the process is controlled by incorporating sulfur compound in the charge mixture at a level lower than the optimum sulfur compound content at zero base content, and adjusting the base content to provide a combination on or near said locus.

**Revendications**

1. Procédé de production d'une arylhydroxylamine représenté par la formule ci-après:

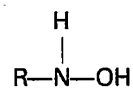

dans laquelle R est un groupe aryle ou aryle substitué, avec un rendement élevé et une formation minimale de composés arylamines sous-produits, caractérisé en ce qu'il comprend les étapes suivantes:
on prépare un mélange de charge comprenant un composé nitroaryle de formule:

$$R{-}NO_2$$

où R est tel que défini plus haut, un catalyseur contenant du platine, une base choisie dans le groupe formé par l'ammoniac, des amines aliphatiques, la phosphine, des phosphines aliphatiques, des arylphosphines et des phosphite esters, un composé du soufre choisi dans le groupe comprenant des composés du soufre divalent dans lesquels le soufre est lié à deux autres parties et des composés réductibles en ces composés du soufre divalent dans des conditions d'hydrogénation catalytiques en un solvant pour le composé nitroaryle, ce solvant étant choisi dans le groupe consistant en alcools, éthers et esters; et
on introduit de l'hydrogène dans ce mélange tout en agitant celui-ci à une température dans une gamme entre 0 et 40°C, de façon à réduire le groupe nitro du composé nitroaryle en groupe hydroxylamine.

2. Procédé suivant la Revendication 1, caractérisé en ce que le composé du soufre est choisi dans le groupe consistant en sulfures et sulfoxydes.

3. Procédé suivant la Revendication 2, caractérisé en ce que le composé du soufre est choisi dans le groupe consistant en dialcoylsulfoxydes et arylalcoylsulfoxydes.

4. Procédé suivant la Revendication 3, caractérisé en ce que le composé du soufre comprend un dialcoylsulfoxyde et que le mélange contient entre 150 et 400 moles de dialcoylsulfoxyde par atome-gramme de platine.

5. Procédé suivant la Revendication 2, caractérisé en ce que le composé du soufre est choisi dans le groupe consistant en sulfures de dialcoyle, disulfures de dialcoyle, sulfures d'aryle et d'alcoyle, hydrogène sulfuré, thiols, thiophène et thiophane.

6. Procédé suivant la Revendication 1, caractérisé en ce que le composé du soufre comprend un sulfure de dialcoyle et que ce mélange contient entre 0,1 et 10 moles de sulfure et de dialcoyle par atome-gramme de platine.

7. Procédé suivant la Revendication 1, caractérisé en ce que la base est choisie dans le groupe consistant en ammoniac et amines aliphatiques.

8. Procédé suivant la Revendication 1, caractérisé en ce que le mélange contient entre $3{,}0.10^{-5}$ et $5{,}0.10^{-4}$ atome-gramme de platine par mole de composé nitroaryle.

9. Procédé suivant la Revendication 4 ou la Revendication 8, caractérisé en ce que la base est choisie dans le groupe consistant en ammoniac et amines aliphatiques et que ce mélange contient entre 100 et 300 moles de base par atome-gramme de platine.

10. Procédé suivant la Revendication 3, caractérisé en ce qu'il y a un maximum de sélectivité du catalyseur pour un lieu géométrique optimum de combinaisons de teneur en composé du soufre et de teneur en base et que l'on contrôle le procédé en incorporant un composé du soufre dans le mélange de charge à un taux inférieur à la teneur optimale en composé du soufre pour une teneur nulle en base et en ajustant la teneur en base pour fournir une combinaison correspondant à ce lieu ou proche de celui-ci.

11. Procédé suivant la Revendication 10, caractérisé en ce que le composé du soufre est du diméthylsulfoxyde et que la base est l'ammoniac, que le diméthylsulfoxyde est incorporé dans le mélange de charge à raison de 200 à 260 moles par atome-gramme de platine et que le taux d'ammoniac est ajusté entre 50 et 300 moles par atome-gramme de platine.

12. Procédé suivant la Revendication 1, caractérisé en ce que le composé nitroaryle est choisi dans le groupe formé par le nitrobenzène, l'o-nitrotoluène, le m-nitrotoluène, le p-nitrotoluène, le p-nitrophénol, le 2,6-diméthylnitrobenzène et l'alpha-nitro-naphtalène.

13. Procédé suivant la Revendication 12, caractérisé en ce que le composé nitroaryle comprend le nitrobenzène.

14. Procédé suivant la Revendication 1, caractérisé en ce que le solvant comprend un alcool primaire.

15. Procédé suivant la Revendication 1, caractérisé en ce que l'hydrogène est introduit dans le mélange tandis que celui-ci est agité à une température dans la gamme allant de 15 à 20°C.

16. Procédé suivant la Revendication 1, caractérisé en ce que cette arylhydroxylamine est convertie en sel d'ammonium d'une N-nitrosoarylhydroxylamine par les étapes suivantes:
on sépare le catalyseur du mélage réactionnel obtenu par hydrogénation du mélange de charge;
ou ajoute de l'ammoniac au mélange réactionnel;
et on ajoute un nitrite d'alcoyle au mélange réactionnel en présence d'ammoniac, de façon à convertir l'arylhydroxylamine en sel d'ammonium de N-nitrosoarylhydroxylamine.

17. Procédé suivant la Revendication 1, caractérisé en ce que le composé du soufre comprend du diméthyl sulfoxyde.

18. Procédé suivant la Revendication 5, caractérisé en ce que le composé du soufre est choisi dans le groupe formé par le sulfure de diméthyle, le sulfure d'éthyle et de propyle, le sulfure de bis(dodécyle) et le sulfure de méthyle et d'octyle.

19. Procédé de production d'une arylhydroxylamine représenté par la formule ci-après:

$$\begin{array}{c} H \\ | \\ R—N—OH \end{array}$$

dans laquelle R est un groupe aryle ou aryle substitué, avec un rendement élevé et une formation minimale de composés arylamines sous-produits, caractérisé par les étapes suivantes:
on prépare un mélange de charge comprenant un composé nitroaryle représenté par la formule:

$$R—N0R—NO_2$$

dans laquelle R est tel que défini plus haut, un catalyseur contenant du platine, une base choisie dans le groupe consistant en ammoniac, amines aliphatiques, phosphine, phosphines aliphatiques, arylphosphines et phosphite esters, un composé du soufre divalent dans lequel le soufre est relié à deux autres parties et un solvant pour le composé nitroaryle, ce solvant étant choisi dans le groupe consistant en alcools, éthers et esters; et
on introduit de l'hydrogène dans ce mélange tout en agitant celui-ci à une température dans la gamme allant de 0 à 40°C, de façon à réduire le groupe nitro du composé nitroaryle en groupe hydroxylamine.

20. Procédé suivant la Revendication 19, caractérisé en ce qu'on a un maximum de sélectivité du catalyseur pour un lieu géométrique optimum de combinaison de la teneur en composé du souf̱fe et de la teneur en base et que l'on contrôle le procédé en incorporant le composé du soufre dans le mélange de charge à un taux inférieur à la teneur optimale en composé du soufre pour une teneúr nulle en base et en ajustant la teneur en base de façon à fournir une combinaison correspondant à ce lieu géométrique ou proche de celui-ci.

**Patentansprüche**

1. Verfahren zur Herstellung eines Arylhydroxylamins der Formel

$$\begin{array}{c} H \\ | \\ R—N—OH \end{array}$$

worin R Aryl oder substituiertes Aryl ist, mit hoher Ausbeute und mit minimaler Bildung von Nebenprodukt-Arylaminverbindungen, dadurch gekennzeichnet, daß das Verfahren die folgenden Stufen umfaßt:
Herstellung eines Beschickungsgemisches, welches eine Nitroarylverbindung der formel

$$R—NO_2$$

worin R wie oben definiert ist, einen Platin enthaltenden Katalysator, eine Base, ausgewählt aus der Gruppe Ammoniak, aliphatische Amine, Phosphin, aliphatische Phosphine, Arylphosphine und Phosphitester, eine Schwefelverbindung, ausgewählt aus der Gruppe zweiwertige Schwefelverbindungen, bei denen Schwefel an zwei weitere Gruppierungen gebunden ist, und Verbindungen, die unter katalytischen Hydrierungsbedingungen zu solchen zweiwertigen Schwefelverbindungen reduzierbar sind, und ein Lösungsmittel für die Nitroarylverbindung, wobei das Lösungsmittel aus der Gruppe Alkohole, Ether und Ester ausgewählt ist, enthält; und
Einführung von Wasserstoff in das Gemisch unter Rühren bzw. Durchbewegen des Gemisches bei einer Temperatur im Bereich von zwischen 0 und 40°C, wodurch die Nitrogruppe der Nitroarylverbindung in die Hydroxylamingruppe reduziert wird.

2. Verfahren nach Anspruch 1, dadurch gekennzeichnet, daß die Schwefelverbindung aus der Gruppe Sulfide und Sulfoxide ausgewählt ist.

3. Verfahren nach Anspruch 2, dadurch gekennzeichnet, daß die Schwefelverbindung aus der Gruppe Dialkylsulfoxide und Arylalkylsulfoxide ausgewählt ist.

4. Verfahren nach Anspruch 3, dadurch gekennzeichnet, daß die Schwefelverbindung ein Dialkylsulfoxid umfaßt und daß das Gemisch zwischen 150 und 400 Mol Dialkylsulfoxid pro Grammatom Platin enthält.

5. Verfahren nach Anspruch 2, dadurch gekennzeichnet, daß die Schwefelverbindung aus der Gruppe Dialkylsulfide, Dialkyldisulfide, Arylalkylsulfide, Schwefelwasserstoff, Thiole, Thiophen und Thiophan ausgewählt ist.

6. Verfahren nach Anspruch 1, dadurch gekennzeichnet, daß die Schwefelverbindung ein Dialkylsulfid um faßt und daß das Gemisch zwischen 0,1 und 10 Mol Dialkylsulfid pro Grammatom Platin enthält.

7. Verfahren nach Anspruch 1, dadurch gekennzeichnet, daß die Base aus der Gruppe Ammoniak und aliphatische Amine ausgewählt ist.

8. Verfahren nach Anspruch 1, dadurch gekennzeichnet, daß das Gemisch zwischen $3{,}0 \times 10^{-5}$ und, $5{,}0 \times 10^{-4}$ Grammatome Platin pro Mol Nitroarylverbindung enthält.

9. Verfahren nach Anspruch 4 oder 8, dadurch gekennzeichnet, daß die Base aus der Gruppe Ammoniak und aliphatische Amine ausgewählt ist und daß das Gemisch zwischen 100 und 300 Mol Base pro Grammatom Platin enthält.

10. Verfahren nach Anspruch 3, dadurch gekennzeichnet, daß es eine maximale Katalysator-Selektivität bei einem optimalen Ort der Kombinationen des Gehalts an Schwefelverbindung und Gehalts an Base gibt und daß man das Verfahren dadurch kontrolliert, daß man die Schwefelverbindung in das Beschickungsgemisch bei einem Niveau einarbeitet, das niedriger ist als der optimale Schwefelgehalt bei einem Null-Gehalt an Base, und daß man den Gehalt der Base so einstellt, daß eine Kombination an oder in der Nähe des genannten Orts erhalten wird.

11. Verfahren nach Anspruch 10, dadurch gekennzeichnet, daß die Schwefelverbindung Dimethylsulfoxid ist und daß die Base Ammoniak ist, daß man das Dimethylsulfoxid in das Beschickungsgemisch mit zwischen 200 und 260 Mol pro Grammatom Platin einarbeitet und daß man den Ammoniakgehalt auf zwischen 50 und 300 Mol pro Grammatom Platin einstellt.

12. Verfahren nach Anspruch 1, dadurch gekennzeichnet, daß man die Nitroarylverbindung aus der Gruppe Nitrobenzol, o-Nitrotoluol, m-Nitrotoluol, p-Nitrotoluol, p-Nitrophenol, 2,6-Dimethylnitrobenzol und α-Nitronaphthalin auswählt.

13. Verfahren nach Anspruch 12, dadurch gekennzeichnet, daß die Nitroarylverbindung Nitrobenzol umfaßt.

14. Verfahren nach Anspruch 1, dadurch gekennzeichnet, daß das Lösungsmittel einen primären Alkohol umfaßt.

15. Verfahren nach Anspruch 1, dadurch gekennzeichnet, daß man Wasserstoff in das Gemisch einführt, während das Gemisch bei einer Temperatur im Bereich zwischen 15 und 20°C gerührt bzw. durchbewegt wird.

16. Verfahren nach Anspruch 1, dadurch gekennzeichnet, daß man das Arylhydroxylamin in das Ammoniumsalz eines N-Nitrosoarylhydroxylamins durch folgende Stufen umwandelt:

Abtrennung des genannten Katalysators von dem Reaktionsgemisch, das durch Hydrierung des Beschickungsgemisches erhalten worden ist;

Zugabe von Ammoniak zu dem Reaktionsgemisch; und

Zugabe eines Alkylnitrits zu dem Reaktionsgemisch in Gegenwart von Ammoniak, wodurch das Arylhydroxylamin in das Ammoniumsalz von N-Nitrosoarylhydroxylamin umgewandelt wird.

17. Verfahren nach Anspruch 1, dadurch gekennzeichnet, daß die Schwefelverbindung Dimethylsulfoxid umfaßt.

18. Verfahren nach Anspruch 5, dadurch gekennzeichnet, daß die Schwefelverbindung aus der Gruppe Dimethylsulfid, Ethylpropylsulfid, Bis-(dodecyl)-sulfid und Methyloctylsulfid ausgewählt ist.

19. Verfahren zur Herstellung eines Arylhydroxylamins der Formel

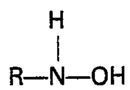

worin R Aryl oder substituiertes Aryl ist, mit hoher Ausbeute und mit minimaler Bildung von Nebenprodukt-Arylaminverbindungen, dadurch gekennzeichnet, daß das Verfahren die folgenden Stufen umfaßt:

Herstellung eines Beschickungsgemisches, das eine Nitroarylverbindung der formel

$$R—NO_2$$

worin R wie oben definiert ist, einen Platin enthaltenden Katalysator, eine Base, ausgewählt aus der Gruppe Ammoniak, aliphatische Amine, Phosphin, aliphatische Phosphine, Arylphosphine und Phosphitester, eine zweiwertige Schwefelverbindung, bei der Schwefel an zwei andere Gruppierungen gebunden ist, und ein

Lösungsmittel für die Nitroarylverbindung, wobei das Lösungsmittel aus der Gruppe Alkohole, Ether und Ester ausgewählt ist, enthält; und

Einführung von Wasserstoff in das Gemisch unter Rühren bzw. Durchbewegen des Gemisches bei einer Temperatur im Bereich von zwischen 0 und 40°C, wodurch die Nitrogruppe der Nitroarylverbindung in die Hydroxylamingruppe reduziert wird.

20. Verfahren nach Anspruch 19, dadurch gekennzeichnet, daß es eine maximale Katalysator-Selektivität bei einem optimalen Ort der Kombinationen des Gehalts an Schwefelverbindung und Gehalts an Base gibt und daß man das Verfahren dadurch kontrolliert, daß man die Schwefelverbindung in das Beschickungsgemisch bei einem Niveau einarbeitet, das niedriger ist als der optimale Schwefelgehalt bei einem Null-Gehalt an Base, und daß man den Gehalt der Base so einstellt, daß eine Kombination an oder in der Nähe des genannten Orts erhalten wird.

SELECTIVITY VS. $NH_3$ LOADING VS. DMSO LOADING